# EUROPÄISCHE PATENTANMELDUNG

(11) **EP 1 051 967 A2**
(43) Veröffentlichungstag der Anmeldung: **15.11.2000**
(21) Anmeldenummer: 00109712.0
(22) Anmeldetag: 08.05.2000
(51) Int. Cl.: A61K 7/48, A61K 7/06

(54) **Kosmetische Zusammensetzung enthaltend modifizierte Stärke**

(30) Priorität: 12.05.1999 DE 19921707
(71) Anmelder: Gräfe Chemie GmbH, 86561 Aresing (DE)
(72) Erfinder: Gräfe, Jürgen E., 86561 Aresing (DE)
(74) Vertreter: Richter, Werdermann & Gerbaulet

(57) **Zusammenfassung**

Kosmetische Zusammensetzung, die als kosmetisch aktiven Bestandteil modifizierte Stärke zusammen mit einem kosmetisch akzeptablen Medium enthält. Insbesondere ist das kosmetisch akzeptable Medium ein oberflächenaktive Substanzen enthaltendes anionisch aufgebautes Shampoo, das zusammen mit der modifizierten Stärke eine klare Löslichkeit aufweist.

## Beschreibung

Die Erfindung betrifft eine kosmetische Zusammensetzung, die als kosmetisch aktiven Bestandteil modifizierte Stärke zusammen mit einem kosmetisch akzeptablen Medium enthält. Insbesondere ist das kosmetisch akzeptable Medium ein oberflächenaktive Substanzen enthaltendes anionisch aufgebautes Shampoo, das zusammen mit der modifierten Stärke eine klare Lösung ergibt.

Die Oberflächeneigenschaften von Haut und Haaren sind in der Kosmetik von besonderem Interesse, und seit jeher besteht ein Interesse daran, kosmetische Produkte zu entwickeln, die Feuchtigkeit spenden und die Oberfläche und das Keratingerüst vorteilhaft beeinflussen. Dies geschieht vielfach dadurch, daß die Wasseraufnahmefähigkeit der oberen Haut- und Keratinschicht verbessert wird. Dafür geeignete Substanzen müssen adhäsive Eigenschaften aufweisen, so daß sie nicht nur anfänglich adsorbiert werden, sondern auch durch Wasser nicht ohne weiteres wieder ausgewaschen werden.

Menschliches Haar besteht aus Keratin, einem schwefelhaltigen fibrillären Protein mit einem isoelektrischen Punkt zwischen pH 3,2 und 4. Das Haar weist somit bei einem in den meisten Pflegemitteln vorhanden pH negative Ladung auf. Es werden deshalb insbesondere kationische Polymere seit langem als Additive in Konditionern und Shampoos oder als separate Behandlung verwendet, um die Kämmbarkeit nasser oder trockener Haare zu verbessern. Die Affinität der kationischen Polymere zu negativ geladenem Haar führt zu einer Filmbildung, welche die Knotenbildung der Haare erschwert und ein Abstehen der Haare durch statische Aufladung beim Kämmen reduziert.

Zu den üblichen bisher in Konditionierern verwendeten Additiven gehören kationische Polymere, wie quaternäre stickstoffhaltige Hydroxyethylcelluloseverbindungen, Kopolymere von Vinylpyrrolidon und Dimethylaminoethylmethacrylat sowie Proteine oder Proteinderivate, wie Kollagen und Kasein und fettige quaternäre Ammoniumverbindungen, wie z.B. Stearyldimethylammoniumchlorid.

Aus der EP 0 521 666 A1 ist die Verwendung ampholytischer Terpolymere in kosmetischen Zusammensetzungen zur Haarbehandlung bekannt. Dabei werden die Eigenschaften der Zusammensetzungen, wie z.B. ihre Interaktion mit den Haaren durch Veränderung der Anteile und Charakteristika der ampholytischen Terpolymere beeinflußt.

Ampholytische Terpolymere, wie zum Beispiel Terpolymere von Acrylamid/Dimethyldiallylammoniumchlorid/Acrylsäure bestehen jedoch nachteilhafterweise aus synthetisch hergestellten Grundstoffen, die teuer in der Herstellung sind. Weiterhin können Probleme in bezug auf die biologische Abbaubarkeit solcher Substanzen auftreten. Außerdem wird die Verwendung künstlicher Stoffe bei der Herstellung von Kosmetika und Körperpflegemitteln von immer weniger Käufern toleriert.

Die Verwendung von modifizierter Stärke als Emulgator und/oder Stabilisator in einer Vielzahl von industriellen Produkten, wie Nahrungsmitteln, Farben, Polymeren, Medikamenten und Kosmetika ist aus dem Stand der Technik bekannt.

So beschreibt zum Beispiel die US 5,185,176 modifizierte Stärke und ihre Verwendung als Emulgator in Nahrungsmitteln.

Die JP 0090278644 AA beschreibt eine Öl/Wasser-Emulsion mit hydrophober metallsalzhaltiger Stärke als Verdickungsmittel und Dispersionsstabilisierer, jedoch nicht als kosmetisch aktiven Bestandteil.

Die JP 0100316546 AA beschreibt ein Haarfärbemittel, das u.a. hydrophob modifizierte Stärke enthält.

Das Lexikon der Hilfsstoffe für Pharmazie, Kosmetik und angrenzende Gebiete (Fiedler, Herbert P.; Bd.2, OVR Oberschwäbische Verlagsanstalt Ravensburg, 1989) beschreibt modifizierte Stärke und ihre Verwendung bei der Tablettenfabrikation als Sprengmittel sowie als Füll- und Gleitmittel, als Salben- und Suppositorienbasis und Auflockerungsmittel für Pasten sowie als Verdickungsmittel, Emulgator, Plasmasubstitut oder Emulsionsstabilisator. Ein Einsatz von modifizierter Stärke in kosmetischen Produkten als aktiver Bestandteil wird nicht beschrieben.

Aus dem Kosmetiklexikon (Burczyk, Aggy; Ehrenwirth Verlag GmbH, München, 1989) sind Stärkederivate als Maskengrundlage und Verdikkungsmittel bekannt. Ihre Verwendung als aktiver Bestandteil in Kosmetika wird nicht beschrieben.

Die WO96/00563 betrifft ein Trockenshampoo unter Mitverwendung pulvriger Stärke.

Die EP 0 550 067 B1 beschreibt eine Verkapselung von Ölen zusammen mit einem Polysaccharid und Emulgatoren durch Sprühtrocknung .

Eine insbesondere zu 5 bis 60 % abgebaute Stärke als Ausgangsmaterial oder/und eine mit Harnstoff, Milchsäure oder/und anderen α-Hydroxysäuren stabilisierte Stärke wird in den oben genannten Dokumenten jedoch weder beschrieben noch nahegelegt.

Modifizierte Stärke zur industriellen Verwendung wird aus pflanzlicher Stärke hergestellt, die in Deutschland insbesondere aus Mais, Kartoffeln und Weizen gewonnen wird. Modifizierte Stärke gehört somit zu den regenerierbaren und biologisch abbaubaren Rohstoffen.

Eine Verwendung von modifizierter Stärke als kosmetisch aktiven Bestandteil von kosmetischen Zusammensetzungen ist aus dem Stand der Technik jedoch weder bekannt noch nahegelegt.

Es ist Aufgabe der vorliegenden Erfindung, verbesserte kosmetische Zusammensetzungen zur Verfügung zu stellen, die fibrillären Faserproteinen und nicht fibrillären Haut-, Haar- und Faserproteinen einen erwünschten Griff und Fülle geben oder wiedergeben, sie pflegen und gleichzeitig gut biologisch abbaubar sind. Die verbesserten kosmetischen Zusammensetzungen müssen verträglich (d.h. insbesondere klar löslich) mit den meisten verwendeten kosmetischen Rohstoffen sein und sich gut verarbeiten lassen. Weiterhin sollen die Herstellungskosten der kosmetischen Zusammensetzungen möglichst gering bleiben.

Diese Aufgabe wird durch die im Anspruch 1 angegebenen Merkmale gelöst.

Die erfindungsgemäßen Zusammensetzungen geben fibrillären Faserproteine und nicht fibrillären Haut-, Haar- und Faserproteinen einen erwünschten Griff und Fülle, pflegen sie und sind biologisch abbaubar. Die verwendete modifizierte Stärke stellt ein kostengünstiges kationisches Polymer da, das aus billigen Rohstoffen gewonnen wird.

Überraschenderweise konnte gefunden werden, daß die modifizierte Stärke als kosmetisch aktiven Bestandteil enthaltenden erfindungsgemäßen kosmetischen Zusammensetzungen strukturverbessernd, glanzverbessernd und hautglättend wirken.

Dies wird auf die stabilitätsverbessernden nichtionischen, anionischen oder kationischen Gruppen der modifizierten Stärke zurückgeführt. Hierdurch entsteht eine besondere Affinität zu den zuvor angegebenen Proteinstrukturen, weil sie adsorptiv an diese binden.

Die erfindungsgemäßen kosmetischen Produkte enthalten als wertbestimmenden Bestandteil Extrakte aus reservekohlenhydrathaltigen Pflanzenteilen, beispielsweise von Mais, Reis, Tapioka oder Kartoffeln. Die Pflanzenteile werden gemahlen oder zerrieben und durch geeignete Maßnahmen aufgeschlossen, so dass die Kohlenhydrate verkleistern und in wässrige Lösung gehen. Im Gegensatz zur chemischen Modifizierung in granulärer Kornstruktur wird die Modifizierung in der wässrigen extrahierten Form durchgeführt, was gravierende Vorteile gegenüber einer Modifizierung an unverkleisterten Stärkekörnern erbringt.

Bei der Modifizierung der granulären Form ist zudem eine Grenze gesetzt, weil ab einem Substitutionsgrad (D.S.) von 0,08 die Stärke stark anquillt, sodass eine Entwässerung wirtschaftlich unvertretbar ist. Demgegenüber ist bei einer Modifizierung in wässrig molekular gelöster Form der Substitutionsgrad beliebig nach oben durchführbar.

Die EP 0 659 394 B1 beschreibt chemisch veränderte Polysaccharide zur Haarverformung (Wasserwelle). Hier wird Stärke in Kornform, also in nicht verkleisterter Struktur mit entsprechenden Reagenzien zur Reaktion gebracht, und dann gemäss Beispiel 1 durch einen Kochprozess in Lösung gebracht oder gemäss Beispiel 2 am intakten Stärkekorn quaternisiert, durch Gelatinierung in eine kationische Form (Quellstärke) überführt. Dieses Verfahren weist den entscheidenden Nachteil auf, dass die Reaktion mehr oder weniger an der Kornoberfläche stattfindet und beim anschließenden Löseprozess auch die inneren nicht oder nur gering modifizierten Polysaccharid-Anteile zwangsläufig anwesend sind. Diese Anteile sind verantwortlich für eine ungenügende Filmbildung. Derartige Produkte schilfern beim Kämmen der Haare ab, erwecken zudem den Eindruck von Schuppenbildung und das Haar wirkt strohig.

Das US-Patent 5,013,763 beschreibt eine Lotion bzw. eine Hautcreme auf Basis von Fetten, Emulgatoren und ggf. einer hydrophoben Stärke. Demgegenüber stellt das erfindungsgemäße Produkt bzw. das erfindungsgemäß hergestellte Produkt ein extrahiertes hydrophiles Produkt dar.

Das US-Patent 5,482,704 beschreibt Öl-in-Wasser-Emulsionen unter Verwendung einer Stärke, die in granulärer Form gemäß Beispiel 1 modifiziert wird, anschließend abgesaugt und getrocknet wird. Für die spätere Verwendung wird diese 1 %ig in Wasser für 20 Minuten intensiv gekocht.

Die JP 0540086629 AA beschreibt eine kosmetische Zusammensetzung unter Zusatz einer kationisch modifizierten Stärke, wobei davon ausgegangen werden muss, dass diese ebenfalls in granulärer Form zur Reaktion gebracht und erst dann in Lösung gebracht wird, was zu den oben beschriebenen Nachteilen führt.

Es ist dabei bevorzugt, wenn das verwendete kosmetische Medium ein anionisch aufgebautes Shampoo oder Körperpflegeprodukt ist und das Stärkederivat gut verträglich ist und klare Löslichkeit ermöglicht.

Als besonders vorteilhaft hat es sich erwiesen, wenn Ausgangsmaterial der modifizierten Stärke eine zu 5 bis 60 %, vorzugsweise zu 10 bis 50 % und am meisten bevorzugt zu 30 bis 40 %, abgebaute Stärke ist. Hierdurch werden die vorteilhaften Eigenschaften der erfindungsgemäßen Zusammensetzung weiter verbessert und hervorragende Pflegeergebnisse bei einer Anwendung erreicht.

Weiterhin als vorteilhaft hat es sich gezeigt, wenn die in der erfindungsgemäßen Zusammensetzung enthaltene Stärke eine mit Harnstoff oder/und Milchsäure oder/und einem Salz davon oder/und anderen α-Hydroxysäuren oder/und Salzen davon stabilisierte Stärke ist. Der Anteil von Harnstoff, Milchsäure oder/und einem Salz davon bzw. anderen α-Hydroxysäuren oder/und Salzen davon an der erfindungsgemäßen Zu-sammensetzung liegt vorteilhafterweise bei:
- Harnstoff:: 0,2 bis 40 %, vorzugsweise 2 bis 20 %,
- Milchsäure:: 0,1 bis 40 %, vorzugsweise 4 bis 20 %,
- andere α-Hydroxysäure:: 0,05 bis 40 %, vorzugsweise 1 bis 20 %.

Besonders bevorzugt ist als α-Hydroxysäure Milchsäure oder/und ein Salz davon. Beispiele für geeignete Salze der Milchsäure sind Na-, K- oder CaLactat. Beispiele für andere geeignete α-Hydroxysäure sind Glykolsäure, Mandelsäure, Äpfelsäure, Weinsäure, Citronensäure etc.

Die Stärke wird durch geeignete Substitutionsgruppen für die jeweilige Anwendung, z.B. als Haar- oder Hautpflegemittel, möglichst optimal ausgestaltet. Der Wert der molekularen Substitution der modifizierten Stärkeprodukte liegt vorzugsweise zwischen 0 und 5.

Durch den kationischen Charakter der modifizierten Stärke und durch die Eigenschaft Hydroxylgruppenbindung einzugehen, sind die Stärkederivate in der Lage, andere Kosmetikformulierungsbestandteile auf der Haut und auf den Haaren zu binden, um ein vorzeitiges Abwandern zu vermeiden. Dies ist wichtig für niedermolekulare Produkte wie z.B. Sonnenschutzmittel, Vitamine und Feuchtigkeitsspendern, deren Wirkung auf der Haut, den Haaren und in den oberen Schichten ablaufen soll.

Bevorzugterweise enthält die erfindungsgemäße kosmetische Zusammensetzung modifizierte Stärke in einer Menge zwischen 0,01 bis 5 Gew.-%, besonders bevorzugt von 0,1 bis 2 Gew.-%.

Weiter bevorzugt sind erfindungsgemäße kosmetische Zusammensetzungen in Form eines 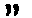 rinse-off"-Produkts, wie z.B. Shampoo, Konditioner, Duschbad, Badeprodukt, flüssige Seife oder Detergens oder eines 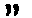 leave-on"-Produkts, wie z.B. Konditioner, Creme oder Lotion. Besonders bevorzugt ist, daß das verwendete kosmetische Medium ein anionisch aufgebautes Shampoo oder Körperpflegeprodukt ist.

Weiterhin konnte gefunden werden, daß die erfindungsgemäßen Stärkederivate gut verträglich mit den meisten bekannten und verwendeten kosmetischen Rohstoffen sind. Bevorzugt sind dabei modifizierte Stärkeprodukte, die zusammen mit dem kosmetisch geeigneten Medium eine klare Lösung ergeben. Die Derivate liegen üblicherweise in flüssiger Form vor, wodurch die Verarbeitung vereinfacht und verbilligt wird. Sie können mit natürlichen, bzw. in der Lebensmittelindustrie verwendeten Konservierungsmitteln konserviert werden.

Eine besonders vorteilhafte Weiterbildung der Erfindung stellt ein Verfahren zur Herstellung einer kosmetischen Zusammensetzung nach Anspruch 1 zur Verfügung, das dadurch gekennzeichnet ist, daß zu einem kosmetisch akzeptablen Medium als kosmetisch aktiver Bestandteil modifizierte Stärke hinzugefügt wird.

Zu einem flüssigen oder pastösen Pflegemittel werden die Stärkederivate bevorzugterweise in Form eines Flüssigprodukts zugesetzt. Man kann die Produkte aber auch in geeigneter Weise trocknen, sie trocken Trocken-produkten zugeben oder erneut in Lösung bringen.

In einer bevorzugten Ausführungsform wird als Ausgangsmaterial der modifizierten Stärke eine zu 5 bis 60 %, vorzugsweise zu 10 bis 50 % und am meisten bevorzugt zu 30 bis 40 %, abgebaute Stärke in das erfindungsgemäße Verfahren eingesetzt.

Weiterhin bevorzugt wird in dem erfindungsgemäßen Verfahren eine mit Harnstoff oder/und Milchsäure oder/und einem Salz davon oder/und anderen α-Hydroxysäuren oder/und Salzen davon stabilisierte Stärke verwendet. Alternativ kann auch gleichzeitig mit der Stärke eine geeignete Menge Harnstoff oder/und Milchsäure oder/und eines Salzes davon oder/und anderer α-Hydroxysäuren oder/und Salzen davon in das erfindungsgemäße Verfahren eingesetzt werden, wodurch die modifizierte Stärke stabilisiert wird. Die Menge dieser Bestandteile wird dabei so gewählt, dass ein guter Stabilisierungseffekt erreicht wird. Vorteilhafterweise beträgt die Menge an Harnstoff 2 bis 15 %, vorzugsweise 5 bis 10 %, bzw. an Milchsäure oder/und einem Salz davon 2 bis 12 %, vorzugsweise 4 bis 8 %, bzw. an anderen α-Hydroxysäuren oder/und Salzen davon 1 bis 15 %, vorzugsweise 4 bis 10 %. Besonders bevorzugt wird als α-Hydroxysäure Milchsäure oder/und ein Salz davon verwendet.

Besonders bevorzugt ist ein erfindungsgemäßes Verfahren, das dadurch gekennzeichnet ist, daß die modifizierte Stärke in einer Menge zwischen 0,01 bis 5 Gew.-%, noch mehr bevorzugt 0,1 bis 2 Gew.-% zu dem kosmetisch akzeptablen Medium hinzugefügt wird.

Weitere zweckmäßige Ausgestaltungen der Erfindung sind in den Unteransprüchen gekennzeichnet.

Eine weitere vorteilhafte Weiterbildung der Erfindung stellt die Verwendung einer modifizierten wie oben beschriebenen Stärke in kosmetischen Zusammensetzungen dar, insbesondere in Shampoos und Körperpflegemitteln.

Besonders vorteilhaft wird erfindungsgemäß eine zu 5 bis 60 % abgebaute modifizierte Stärke verwendet. Schließlich kann die verwendete Stärke mit Harnstoff oder/und Milchsäure oder/und einem Salz davon oder/und anderen α-Hydroxysäuren oder/und Salzen davon stabilisiert sein. Alternativ können diese Bestandteile einzeln oder in Kombination zusammen mit der modifizierten Stärke in einer für eine ausreichende Stabilisierung der Stärke sorgenden Menge verwendet werden.

Die Erfindung soll im folgenden näher beschrieben werden.

Zur Herstellung einer erfindungsgemäßen kosmetischen Zusammensetzung, z.B. eines anionisch aufgebauten Shampoos, wird zu einem flüssigen oder pastösen Pflegemittel auf an sich bekannte Weise modifizierte Stärke zugesetzt. Dabei liegt der Wert der molekularen Substitution der zugesetzten modifizierten Stärkeprodukte idealerweise zwischen 0 und 5. Üblicherweise wird die modifizierte Stärke in einer Menge zwischen 0,01 bis 5 Gew.-% des Pflegemittels zugegeben. Dabei wird darauf geachtet, dass das modifizierte Stärkeprodukt zusammen mit dem kosmetisch geeigneten Medium eine klare Lösung ergibt.

Die in den erfinderischen Mitteln zur Verwendung kommenden Ausgangsprodukte sind natürlichen Ursprungs aus pflanzliche Quellen und werden zunächst aus diesen extrahiert. Zu den zur Durchführung der Erfindung geeigneten Ausgangsstoffen zählen alle reservekohlenhydrathaltigen Pflanzenteile, beispielsweise die von Mais, Reis, Weizen, Tapioka oder Kartoffeln.

Die Pflanzenteile werden gemahlen oder zerrieben und durch geeignete Maßnahmen aufgeschlossen, so daß die Kohlenhydrate verkleistern und in wäßrige Lösung gehen. Für die weitere Verwendung ist dann eine Filtration unbedingte Voraussetzung, um so die extrahierten Stoffe vom Ungelösten zu trennen.

Alternativ kann man die käuflichen Extrakte, beispielsweise Stärke aus Mais, Reis, Weizen, Tapioka oder Kartoffeln einsetzen und diese in entsprechender Weise aufschließen und in Lösung bringen.

Die modifizierte Stärke kann in Form eines Flüssigprodukts zugesetzt werden. Man kann die Produkte aber auch in geeigneter Weise trocknen, sie trocken Trockenprodukten zugeben oder erneut in Lösung bringen.

Auch die Verwendung von Mehlen aus Mais, Reis, Weizen, Tapioka oder Kartoffeln und anderen kohlenhydrathaltigen Pflanzen oder Pflanzenteilen ist in diese Erfindung einbezogen.

Zusätzlich können natürliche Proteine und/oder lösliche Mineralstoffe zugesetzt werden. Dies ist eine Alternative, aber keine Mussvorgabe.

Die so erhaltenen kosmetischen Zusammensetzungen können dann je nach Anwendungszweck auf an sich bekannte Weise entsprechend weiter verarbeitet werden.

Bei dem Vorgehen treten in der erhaltenen Lösung Stabilitätsprobleme auf, die unter dem Begriff Retrogradation bekannt sind. Um die Nachteile zu eliminieren, werden die pflanzlichen Extrakte vorzugsweise stabilisiert. Dies erfolgt in bekannter Weise durch molekularen Abbau durch Oxidationsmittel, Säuren, Enzymen und/oder durch Addition von Ester- und/oder Ethergruppen in bekannter Weise, wobei nichtionische, anionische bzw. kationische oder auch bi-ionische Derivate zugänglich sind und der Substitutionsgrad (DS) so gehalten wird, daß noch eine biologische Abbaufähigkeit gewährleistet ist.

Überraschenderweise zeigten sich besonders gute Stabilitäten der Lösungen bzw. der Inhaltsstoffe, wenn Harnstoff und Milchsäure oder/und ein Salz davon oder/und andere α-Hydroxysäuren oder/und Salze davon zugesetzt wurde. Als besonders vorteilhaft hat sich eine Menge von 2 bis 25 % dieser Mischung, als noch vorteilhafter von 4 bis 20 % und am vorteilhaftesten von 5 bis 15 % erwiesen. Insbesondere der Einsatz von mit Harnstoff und Milchsäure oder/und einem Salz davon oder/und anderen α-Hydroxysäuren oder/und Salzen davon stabilisierter Stärke hat vorteilhafte Stabilitätseigenschaften derartiger Lösungen erzielt.

Bei der Haarbehandlung wirken die erfindungsgemäßen Zusammensetzungen positiv, indem sie Haut und Haare pflegen, dem Haar Body (Körper) geben ohne zu kleben und als Konditionierer wirken.

Bei der Verwendung der erfindungsgemäßen Stärkederivate tritt als zusätzlicher positiver Effekt ein weicher und kremiger Schaum auf, der eine feine und sahnige Konsistenz hat. Weiterhin erfolgt ein schnelles Anschäumen, der Schaum ist leicht auszuspülen, gut zu verteilen und angenehm an den Händen.

Die Haare sind nach einer Behandlung mit den Stärkederivaten leichter weiter zu behandeln, insbesondere bei Dauerwellen, Blondierung, Färben, Entkräuselung oder Entfernung von Dauerwellen.

Das Haar wird geschmeidiger, besonders volles Haar behält seine Struktur, bleibt voluminös und behält seine Form. Die erfindungsgemäßen Zusammensetzungen wirken weiterhin der Austrocknung der Haut und der Haare entgegen, verbessern die Naß- und Trockenkämmbarkeit, und wirken der Knotenbildung besonders langer Haare entgegen.

Die Stärkederivate in den erfindungsgemäßen Zusammensetzungen bilden durch ihre kationische Ladung und durch Hydroxylgruppenbindung mehr oder weniger starke Filme auf der Haut und den Haaren, da diese negativ geladen sind. Da diese Filme hygroskopischen Charakter haben, versorgen sie Haut und Haare mit Feuchtigkeit und verhindern so eine Austrocknung. Als besonders vorteilhaft hat es sich erwiesen, zu 5 bis 60 % abgebaute Stärkederivate zu verwenden. Herkömmlich verwendete kationische Polymere neigen zum sogenannten 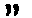 built-up" und sind durch einfaches Waschen nicht zu entfernen. Auch bei dauerhafter Anwendung konnte keine Anreicherung der Stärkederivate der erfindungsgemäßen Zusammensetzungen beobachtet werden.

Eine Penetration der Stärkederivate in die oberen Hautschichten mit anschließender Stärkung des Keratingerüstes der Haare ist auf Grund des niedrigen Molekulargewichts anzunehmen.

Bei der Körperpflege wirken die erfindungsgemäßen Zusammensetzungen positiv, indem sie ein angenehmes weiches Hautgefühl hinterlassen, nicht kleben und einen feuchtigkeitsbindenden und -spenden Film auf der Haut bilden.

Die Haut fühlt sich seidig an. Die Haut wird angenehm weich und vor Austrocknung geschützt, ohne zu fetten.

Die erfindungsgemäßen Zusammensetzungen geben fibrillären Faserproteinen und nicht fibrillären Haut-, Haar- und Faserproteinen einen erwünschten Griff und Fülle, pflegen sie und sind biologisch abbaubar.

Der Begriff Faserproteine umfaßt Pelze und Felle, also tierische Skleroproteine mit Faserstruktur, insbesondere Wolle und Seide, sowie Haare bzw. Haarfasern menschlichen und tierischen Ursprungs ganz allgemein, sowie Haut- und Hautanhangsgebilde.

Die in den erfinderischen Mitteln zur Verwendung kommenden Ausgangsprodukte sind natürlichen Ursprungs aus pflanzliche Quellen und werden aus diesen extrahiert. Zu den zur Durchführung der Erfindung geeigneten Ausgangsstoffen zählen alle reservekohlenhydrathaltigen Pflanzenteile, beispielsweise die von Mais, Reis, Weizen, Tapioka oder Kartoffeln.

Die Pflanzenteile werden gemahlen oder zerrieben und durch geeignete Maßnahmen aufgeschlossen, so daß die Kohlenhydrate verkleistern und in wäßrige Lösung gehen.

Erfindungsgemäß kann man alternativ die käuflichen Extrakte, beispielsweise Stärke aus Mais, Reis, Weizen, Tapioka oder Kartoffeln einsetzen und diese in entsprechender Weise aufschließen und in Lösung bringen.

Besonders gute Ergebnisse wurden insbesondere mit modifizierter Stärke erreicht, für die als Ausgangsstoff eine zu 5 bis 60 % abgebaute Stärke verwendet wurde. Der Abbaugrad kann mit Mitteln erreicht werden, die dem Fachmann bekannt sind und sie werden deshalb hier nicht näher ausgeführt.

Auch die Verwendung von Mehlen aus Mais, Reis, Weizen, Tapioka oder Kartoffeln und anderen kohlenhydrathaltigen Pflanzen oder Pflanzenteilen ist in diese Erfindung einbezogen.

Die neuen Behandlungsmittel wirken strukturverbessernd, ggf. glanzverbessernd und hautglättend, was auf die stabilitätsverbessernden nichtionischen, anionischen oder kationischen Gruppen zurückgeführt wird. Hierdurch entsteht eine besondere Affinität zu den zuvor angegebenen Proteinstrukturen, weil sie adsorptiv an diese binden.

Folgende Formulierungen sind bereits mit vorteilhaften Ergebnissen eingesetzt worden:

### Creme-Spül-Konditionierer

| Position | Rohstoffe | % |
|---|---|---|
| 1. | Paraffinum Liquidum | 2,00 |
| 2. | Isopropylpalmitat | 1,00 |
| 3. | Ceterylalcohol | 3,00 |
| 4. | Cetrimoniumchlorid | 1,00 |
| 5. | Modifizierte Kartoffelstärke | 0,80 |
| 6. | PEG-3 Lauraminoxid | 1,00 |
| 7. | Zitronensäure bis pH 5,5 | q.s. |
| 8. | Duftstoffe, Konservierungsmittel | q.s. |
| 9. | Wasser zu | 100,00 |

Auch stark gestreßtes (chemisch behandeltes und langes Haar) ließ sich nach der Behandlung leicht nass und trocken kämmen. Das Haar fiel leicht und locker und fühlte sich sehr weich an. Der 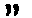 softening" Effekt war besonders bei der kationischen Form sehr ausgeprägt.

### Conditioning Shampoo

| Position | Rohstoffe | % |
|---|---|---|
| 1. | Ammoniumlaurylsulfat | 18,00 |
| 2. | Ammoniumlaurethsulfat | 18,00 |
| 3. | Glycolstearat | 3,00 |
| 4. | Cocoamid MEA | 2,50 |
| 5. | Ceterylalcohol | 1,00 |
| 6. | Carbomer 934 | 1,50 |
| 7. | Triethanolamin | 2,00 |
| 8. | Modifizierte Kartoffelstärke | 0,65 |
| 9. | Wasser | 51,00 |
| 10. | Konservierungsmittel, Duftstoffe, Zitronensäure | q.s. |
| | ph-Wert | 6,0 |

Der alleinige Zusatz der modifizierten Kartoffelstärke in der nichtionischen und in der kationischen Form (DS 0,5) machten das Haar leichter nass und trocken kämmbar. Der sonst strohige Charakter des Haares nach der Wäsche war vollkommen aufgehoben. Die Knotenbildung langer Haare kaum feststellbar, das Haar war leicht zu bearbeiten und im Gefühl weich.

### Conditioning Shampoo

| Position | Rohstoffe | % |
|---|---|---|
| 1. | Na-Laurethsulfat | |
| | Mg-Laurethsulfat | |
| | Na-Laureth-8-sulfat | |
| | Mg-Laureth-8-sulfat | |
| | Na-Olethsulfat | |
| | Mg-Olethsulfat | 20,00 |
| 2. | Cocoamidopropylbetain | 11,00 |
| 3. | Dimethicon | 1,00 |
| 4. | Dimethicon-Copolyolacetat | 1,00 |
| 5. | Modifizierte Kartoffelstärke | 0,60 |
| 6. | Na-Laurethsulfat, Cocoamid-DEA, Glycoldistearat | 10,00 |
| 7. | NaCl | q.s. |
| 8. | Wasser zu | 100,00 |
| 9. | Konservierungsmittel, Duftstoffe | q.s |
| | ph-Wert | 6,5 |

Beide Formen der modifizierten Kartoffelstärke in der nichtionischen und in der kationischen Form (DS 0,5) machten das Haar leichter nass und trocken kämmbar.

Besonders die Enden langer Haare waren knotenfrei und problemlos zu kämmen. Das Haar hafte 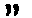 Body" und fühlte sich extrem weich an.

Stark chemisch gestreßtes Haar ließ sich nach der Behandlung leichter kämmen und wirkte leicht und locker.

### Duschbad mit Pflegeadditive

| Position | Rohstoffe | % |
|---|---|---|
| 1. | Coco-Glucosid | 9,00 |
| 2. | Na-Laurethsulfat | 21,00 |
| 3. | Dinatrium Laurethsulfosuccinat | 4,00 |
| 4. | Laurylglucosid (und) -glycol Distearat (und) Glycerin | 5,50 |
| 5. | Hydrolisiertes Weizengluten | 2,00 |
| 6. | Modifizierte Kartoffelstärke | 0,50 |
| 7. | Laureth-2 | 1,50 |
| 8. | NaCl | 3,00 |
| 9. | Wasser zu | 100,00 |
| 10. | Konservierungsmittel, Duftstoffe | q.s. |
| 11. | ph-Wert | 5,2 |

Der pflegende Charakter dieser Formulierung wurde durch den Zusatz der modifizierten Kartoffelstärke in der nichtionischen und in der kationischen Form (DS 0,5) weiter verstärkt. Die Haut fühlte sich glatter an und wirkte besser durchfeuchtet ohne zu kleben. In dieser Formulierung war kein Unterschied in der Wirksamkeit beider Produkte subjektiv feststellbar.

### Duschgel

| Position | Rohstoffe | % |
|---|---|---|
| 1. | Na-Laurethsulfat | 33,50 |
| 2. | Coco-Betain | 10,00 |
| 3. | PPG-5 Laureth-5 | 4,00 |
| 4. | Dimethiconcopolyol | 4,00 |
| 5. | Cocoamid-DEA | 3,50 |
| 6. | Modifizierte Kartoffelstärke | 0,50 |
| 7. | Wasser zu | 100,00 |
| 8. | Konservierung, Duftstoffe, Zitronensäure | q.s. |
| | pH-Wert | 5,5 |

Auch in dieser Formulierung gab die modifizierte Kartoffelstärke in der nichtionischen und in der kationischen Form (DS 0,5) dem Duschgel ein weiches Hautgefühl und hinterließ ein gepflegtes Wohlbefinden, ohne zu kleben. Die Haut wirkte weit weniger trocken. Die Wirkung der kationischen Form war ausgeprägter.

### Duschgel

| Position | Rohstoffe | % |
|---|---|---|
| 1. | Na-Myrethsulfat | 40,00 |
| 2. | Laureth-2 Amid-MEA | 2,00 |
| 3. | Cocoamidopropylbetain | 10,00 |
| 4. | Trideceth-9, PEG-5 Octanoat | 1,00 |
| 5. | Modifizierte Kartoffelstärke | 0,40 |
| 6. | Konservierungsmittel, Duftstoffe | q.s. |
| 7. | NaCl | 1,50 |
| 8. | Zitronensäure | 0,30 |
| 9. | Wasser zu | 100,00 |
| | pH-Wert | 6,0 |

Die modifizierte Kartoffelstärke in der nichtionischen und in der kationischen Form (DS 0,5) vermittelte dem Duschgel ein spürbar verbessertes Hautgefühl. Die Haut fühlte sich weicher, samtseidig an. Die modifizierte Kartoffelstärke sorgte für eine anhaltende Feuchthaltung der Haut. Bei gleicher Konzentration war die kationische Form wirksamer.

### Erfindungspemäße kationische Form einer Stärke (100 % Trockenmasse)

| Position | Rohstoffe | Teile |
|---|---|---|
| 1. | Stärke | 60 |
| 2. | NaOH | 3 - 10 |
| 3. | kationisches Reagenz | 5 - 25 |
| 4. | Harnstoff | 2 - 20 |
| 5. | Milchsäure oder Na-Lactat | 2 - 15 |

Die Wassermenge wird nach gewünschtem Trockenmassegehalt gewählt. Jedes dem Fachmann bekannte und mit den übrigen Bestandteilen verträgliche kationische Reagenz ist für die Erfindung geeignet.

## Patentansprüche

1. Kosmetische Zusammensetzung, dadurch gekennzeichnet, dass sie als kosmetisch aktiven Bestandteil modifizierte Stärke zusammen mit einem kosmetisch akzeptablen Medium enthält.

2. Kosmetische Zusammensetzung nach Anspruch 1, dadurch gekennzeichnet, dass das Ausgangsmaterial der modifizierten Stärke eine zu 5 bis 60 % abgebaute Stärke ist.

3. Kosmetische Zusammensetzung nach Anspruch 1 oder 2, dadurch gekennzeichnet, dass die Stärke eine mit Harnstoff oder/und Milchsäure oder/und einem Salz davon oder/und anderen α-Hydroxysäuren oder/und Salzen davon stabilisierte Stärke ist.

4. Kosmetische Zusammensetzung nach einem der Ansprüche 1 bis 3, dadurch gekennzeichnet, dass sie modifizierte Stärke in einer Menge zwischen 0,01 bis 5 Gew.-% enthält.

5. Kosmetische Zusammensetzung nach einem der Ansprüche 1 bis 4, dadurch gekennzeichnet, dass der Wert der molekularen Substitution der modifizierten Stärke zwischen 0 und 5 liegt.

6. Kosmetische Zusammensetzung nach einem der Ansprüche 1 bis 5, dadurch gekennzeichnet, dass die modifizierte Stärke nichtionisch, anionisch oder kationisch aufgebaut ist.

7. Kosmetische Zusammensetzung nach einem der Ansprüche 1 bis 6, dadurch gekennzeichnet, dass sie ein 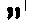 rinse-off"-Produkt, wie z.B. Shampoo, Konditioner, Duschbad, Badeprodukt, flüssige Seife oder Detergenz oder ein 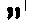 leave-on"-Produkt, wie z.B. Konditioner, Creme oder Lotion ist.

8. Kosmetische Zusammensetzung nach einem der Ansprüche 1 bis 7, dadurch gekennzeichnet, dass das verwendete kosmetisch akzeptable Medium ein anionisch oder nichtionogen aufgebautes Shampoo oder Körperpflegeprodukt ist.

9. Kosmetische Zusammensetzung nach einem der Ansprüche 1 bis 8, dadurch gekennzeichnet, dass das modifizierte Stärkeprodukt zusammen mit dem kosmetisch akzeptablen Medium eine klare Lösung ergibt.

10. Verfahren zur Herstellung einer kosmetischen Zusammensetzung nach Anspruch 1, dadurch gekennzeichnet, dass als kosmetisch aktiver Bestandteil modifizierte Stärke zu einem kosmetisch akzeptablen Medium hinzugefügt wird.

11. Verfahren zur Herstellung einer kosmetischen Zusammensetzung nach Anspruch 10, dadurch gekennzeichnet, dass als Ausgangsmaterial der modifizierten Stärke eine zu 5 bis 60 % abgebaute Stärke hinzugefügt wird.

12. Verfahren zur Herstellung einer kosmetischen Zusammensetzung nach Anspruch 10 oder 11, dadurch gekennzeichnet, dass eine mit Harnstoff oder/und Milchsäure oder/und einem Salz davon oder/und anderen α-Hydroxysäuren oder/und Salzen davon stabilisierte modifizierte Stärke hinzugefügt wird.

13. Verfahren zur Herstellung einer kosmetischen Zusammensetzung nach einem der Ansprüche 10 bis 12, dadurch gekennzeichnet, dass die modifizierte Stärke in einer Menge zwischen 0,01 bis 5 Gew.-% zu dem kosmetisch akzebtablen Medium hinzugefügt wird.

14. Verfahren zur Herstellung einer kosmetischen Zusammensetzung nach einem der Ansprüche 10 bis 13, dadurch gekennzeichnet, dass der Wert der molekularen Substitution der modifizierten Stärkeprodukte zwischen 0 und 5 liegt.

15. Verfahren zur Herstellung einer kosmetischen Zusammensetzung nach einem der Ansprüche 10 bis 14, dadurch gekennzeichnet, dass die modifizierte Stärke nichtionisch, anionisch oder kationisch aufgebaut ist.

16. Verfahren zur Herstellung einer kosmetischen Zusammensetzung nach einem der Ansprüche 10 bis 15, dadurch gekennzeichnet, dass das modifizierte Stärkeprodukt zusammen mit dem kosmetisch akzeptablen Medium eine klare Lösung ergibt.

17. Verfahren zur Herstellung einer kosmetischen Zusammensetzung nach einem der Ansprüche 10 bis 16, dadurch gekennzeichnet, dass die modifizierte Stärke in Form eines Flüssigprodukts zugesetzt wird.

18. Verwendung von modifizierter Stärke in kosmetischen Zusammensetzungen.

19. Verwendung nach Anspruch 18, dadurch gekennzeichnet, dass die modifizierte Stärke eine zu 5 bis 60 % abgebaute Stärke ist.

20. Verwendung nach Anspruch 18 oder 19, dadurch gekennzeichnet, dass die modifizierte Stärke eine mit Harnstoff oder/und Milchsäure oder/und einem Salz davon oder/und anderen α-Hydroxysäuren oder/und Salzen davon stabilisierte Stärke ist.
